# EUROPEAN PATENT APPLICATION

(11) **EP 1 774 935 A1**
(43) Date of publication of application: **18.04.2007**
(21) Application number: 05755172.3
(22) Date of filing: 29.06.2005
(51) Int. Cl.: A61F 13/15

(54) **PAPER DIAPER**

(30) Priority: 30.06.2004 JP 2004194853; 29.11.2004 JP 2004344715; 08.02.2005 JP 2005031662; 31.03.2005 JP 2005103856
(71) Applicant: DAIO PAPER CORPORATION, Shikokuchuo-shi, Ehime 799-0492 (JP)
(72) Inventor: KAMOTO, Tomoka, c/o DAIO PAPER CONVERTING CO., LTD, Shikokuchuo-shi, Ehime 7990431 (JP); KUBO, Taira, c/o DAIO PAPER CONVERTING CO., LTD., Shikokuchuo-shi, Ehime 7990431 (JP); MIYASHITA, Yoshiharu c/o DAIO PAPER CONVERT.CO.LTD, Shikokuchuo-shi, Ehime 7990431 (JP); MATSUI, Tomotsugu, DAIO PAPER CONVERTING CO., LTD., Shikokuchuo-shi, Ehime 7990431 (JP); HANAO, Hiroyuki c/o DAIO PAPER CONVERTING CO., LTD, Shikokuchuo-shi, Ehime 7990431 (JP)
(74) Representative: Tollett, Ian
(86) International application number: PCT/JP2005/011961
(87) International publication number: WO 2006/003942

(57) **Abstract**

[Problem to be Solved] To prevent shingly hand feeling and unintentional bias of absorption characteristics in the case of using tow (fiber bundle).

[SOLUTION] An absorption element having a super absorbent polymer 54, comprising a fiber aggregate formed by opening tow 21, a super absorbent polymer 54 and a wrapping sheet 58 for wrapping them, wherein the whole face or almost whole face of part provided at least with the super absorbent polymer 54 in the sheet 58 is applied with an adhesive in a continuous plane, which is bonded to the sheet 58 with the adhesive.

## Description

### [Field of the Invention]

The present invention relates to a disposable diaper with improved absorption and retention performance of loose stool.

### [Prior Art]

In disposable diapers there has been so far a problem on the absorption and retention performance of loose stool. Specifically, there are instances that loose stool remains on a top sheet not passing through the top sheet, or loose stool flows back onto a top sheet without retaining loose stool which has passed through the top sheet in an absorber to cause skin fit or to need frequent works of skin wiping.

From such viewpoints, there has been proposed a technique that a perforated sheet is used as a top sheet to pass loose stool easily in order to improve capability of accommodating and retaining loose stool at a far distance from skin (simply also referred to as absorption performance of loose stool etc.) as well as keep loose stool at a distance from skin quickly, and a technique of forming an accommodation space of loose stool between a top sheet and an absorber (e.g., see Japanese Unexamined Patent Application Publication Hei 2-65861 (1990)).

However, with intensified technical competitions in recent years, further improvements are presently desired regarding absorption and retention performance of loose stool.
[Patent document 1] Japanese Unexamined Patent Application Publication Hei 2-65861 (1990)

### [Disclosure of the Invention]

### [Problems to be solved by the Invention]

Therefore, a primary object of the present invention is to solve the above problems.

### [Means to solve the Problems]

The present invention that has solved the above problems is a disposable diaper comprising a lower layer absorber and an upper layer absorber provided thereon, wherein the upper layer absorber has passage holes of loose stool, and at least one of the lower layer absorber and the upper layer absorber is formed by a fiber aggregate formed by opening tow.

Namely, the present invention proposes a disposable diaper that at least the lower layer absorber is formed by a fiber aggregate formed by opening tow, in addition thereto, one that at least the upper layer absorber is formed by a fiber aggregate formed by opening tow.

In the present invention, since an upper layer absorber has a passage hole with a wider diameter than fiber distance, loose stool is allowed to pass through the passage hole to be quickly kept at a distance from skin. Thereafter, at least one of the lower layer absorber and the upper layer absorber is formed by a fiber aggregate formed by opening tow, which brings the following merits. Namely, in the case of fiber aggregate where a lower layer absorber is formed by a fiber aggregate formed by opening tow, the lower layer absorber is excellent in ease of expandability (or resilience) in absorption of loose stool, and also absorbs loose stool quickly and definitely due to high absorption speed of liquid and residual solid content is firmly retained without its flow back. Also, in the case of fiber aggregate where an upper layer absorber is formed by a fiber aggregate formed by opening tow, since it is excellent in shape retention of passage hole and cushioning property due to long fiber length, the lower layer absorber that has absorbed and retained loose stool can be firmly kept at a far distance from skin.

A disposable diaper has generally a mode that an absorber is interposed between a top sheet and a body liquid impermeable sheet. In the case of application in such mode, it has preferably a top sheet of perforated sheet capable of passing loose stool and a body liquid impermeable sheet, and the foregoing upper layer absorber and lower layer absorber interposed between the top sheet and body liquid impermeable sheet. By a top sheet formed by a perforated sheet capable of passing loose stool, loose stool and the like pass through quickly into absorber side, further pass quickly trough the passage holes of upper layer absorber into lower layer absorber side, and absorbed and retained by the lower layer absorber.

As a fiber aggregate formed by opening tow, depending on fineness and material of fiber aggregate, and bonding level of fibers each other, in general, use of fiber density of 0.0075 g/cm³ or less in the thickness of 10 mm is advantageous for improvement of absorption performance by the present invention.

Also, it is preferable that a fiber aggregate formed by opening tow contains super absorbent polymer particles, in this case, a holding sheet is preferably provided in the back surface side of lower layer absorber. In the case where super absorbent polymer particles are contained in a fiber aggregate formed by opening tow, when back surface side of a product is touched, concave-convex clump of SAP particles fallen from fiber aggregate or in under part of fiber aggregate yields shingly uncomfortable feeling, lowering the product value. In contrast, in the case where a holding sheet is provided, such shingly uncomfortable feeling when touched from back surface side of product is reduced or not generated.

### [Effect of the Invention]

As described above, merits of a disposable diaper having excellent absorption performance of loose stool are provided according to the present invention.

### [Best Mode for Carrying Out of the Invention]

An embodiment of the present invention will be described in detail with reference to disposable diapers and the production equipment thereof shown in the attached drawings below.

### <Example of underpants-type disposable diaper>

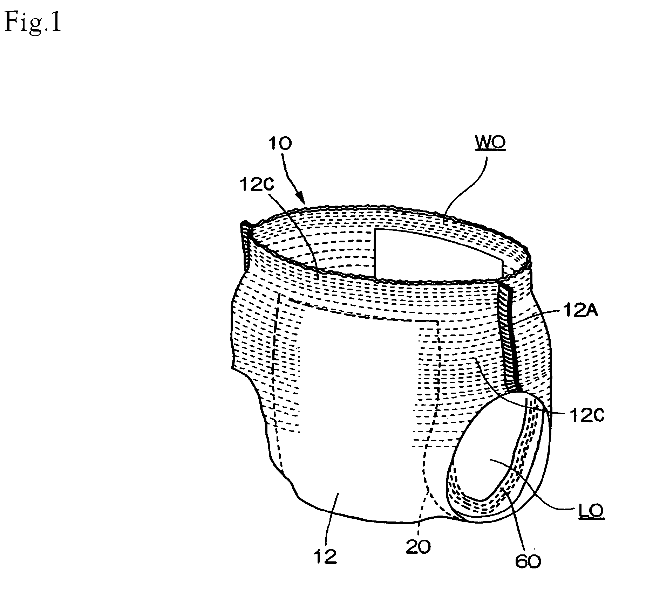

FIG. 1 shows an example of underpants-type disposable diaper. This underpants-type disposable diaper 10 is provided with an external sheet 12 of outer face (back surface) side and an absorptive body 20 of inner face (front face) side, and the absorptive body 20 is fixed with the external sheet 12. The absorptive body 20 is a part to receive body fluids such as urine and loose stool (menstrual blood in sanitary napkin described below), adsorb and retain them. The external sheet 12 is a part to wear a wearer.

The external sheet 12 is shaped like a sand clock as shown in the figure for example, the both sides are narrowed for a wearer to put legs. The absorptive body 20 can shape any form, in the figure, it is rectangular.

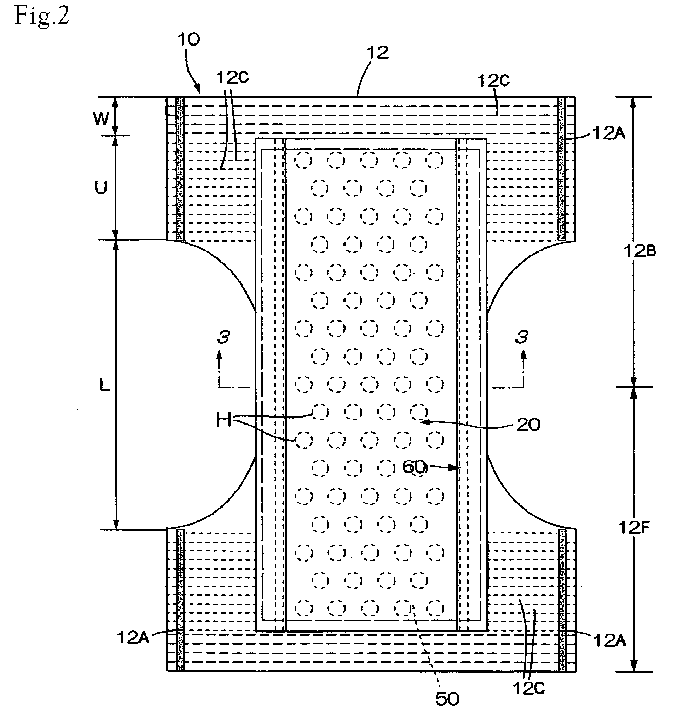

As shown in FIG. 2, the external sheet 12 is folded back and forth after the absorptive body 20 is set and fixed in a given place, a bonding region 12A is bonded by thermal bonding at both sides of a front body 12F and a back body 12B in the external sheet 12. In this way, an underpants-type disposable diaper is obtained having a waist opening WO and a pair of leg openings LO in a structure shown in FIG. 1.

The width in the middle of absorptive body 20 shown in the figure in the longitudinal direction (namely in the up and down direction in FIG. 2, also, back and forth direction of product) is shown as a shape shorter than the width fastening the narrow part of external sheet 12. This relationship of width may be reverse or may be the same width.

The external sheet 12 is desirably constructed with two pieces of water repellent non-woven sheets for example, a shape is desirable such that an elastic member is interposed between these sheets to fit a wearer by the contractive force. As the elastic member, rubber thread and strip material like elastic foam can be used, use of many rubber threads is preferable. In the mode shown in the figure, rubber threads 12C are continuously provided in the width direction in a waist region W, they are provided only in both sides in a hip region U, and not provided in a crotch region L. Since the rubber threads 12C are provided in the waist region W and hip region U, even if rubber thread 12 itself is weak in compressive force, a product fits well to a wearer because they touch a wearer in hip region U as a whole.

### (Absorptive body)

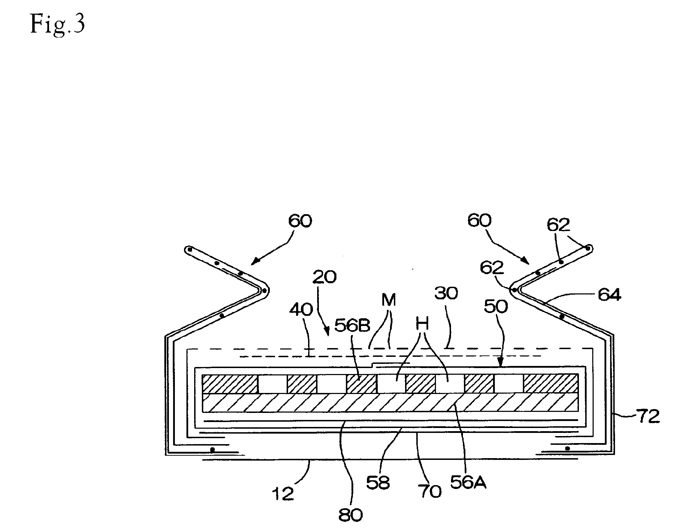

As shown in FIG. 3, an absorptive body 20 in an embodiment is provided with a top sheet 30 composed of non-woven fabric permeating body fluids for example, a medium sheet (second sheet) 40 and an absorption element 50 including an absorber 56. Also, at the back surface side of absorption element 50, a body liquid impermeable sheet (also called back sheet) 70 composed of plastic sheet etc. is provided. On the back surface side of this body fluid impermeable sheet 70, an external sheet 12 is provided. Further, barrier cuffs 60 are provided in both sides.

### (Top sheet)

A top sheet 30 has a property permeating body fluids. Thus, material for the top sheet 30 is any one exhibiting permeability to body fluid, for example, there can be exemplified porous or nonporous non-woven fabric, porous plastic sheets of polyethylene etc, and net materials of plain weave of nylon and polyethylene terephtahalate fibers. Also, raw fibers of the non-woven fabric are not particularly limited. For example, there can be exemplified synthetic fibers such as olefin type like polyethylene and polypropylene, polyester type and polyamide type; regenerated fibers such as rayon and cupra; natural fibers such as cotton; and mixed fibers used in 2 or more kinds thereof. Further, non-woven fabric may be produced in any processing. As the processing method, for example, there can be listed known methods such as spun bond method, air-through method, point bond method, spun lace method, spun bond method, thermal bond method, melt blown method and needle punch method. For example, spun lace method is a preferable processing method for flexibility and drape property, and thermal bond method is a preferable processing method for bulkiness and softness.

Also, the top sheet 30 may be constructed with one sheet, or a laminated sheet consisting of two or more sheets. Similarly, the top sheet 30 may constructed with one sheet or, two or more sheets in regard to the plane direction. For example, a laminate non-woven fabric such as SMS non-woven, or a laminate non-woven fabric laminated with plastic film and non-woven can be used.

As the top sheet 30, a sheet with hole M capable of passing the solid content of loose stool sufficiently, for example, a perforated sheet that a lot of holes M are opened on a non-woven fabric is preferable. In this case, open area ratio of hole M is preferably 10 to 40%, particularly preferably 15 to 20%. The shape of hole M is not particularly limited, it can be suitably selected from circle, triangle, square, lozenge and the like. The holes M can be disposed entirely in a overlapped part of absorbers 3A, 3B, also in a part, for example, can be disposed only in excretion region (almost middle part). Diameter of a hole M is preferably 0.5 to 30 mm, particularly preferably 2 to 5 mm. Further, number density of holes M is preferably 5 to 20 pieces /cm², particularly preferably 6 to 12 pieces /cm². The number density may be uniform in the part containing holes M, or may be changed. Such hole M can be formed by punching-out processing. In the case that there is a problem of insufficient strength due to the size or the number of holes M, it is preferable to set the basis weight of top sheet 30 to be 15 to 50 g/m².

### (Medium sheet)

To transport body fluids passed through top sheet 30 into an absorption element quickly, a medium sheet 40 ordinarily called "second sheet" whose permeation velocity is faster than that of top sheet 30 can be disposed. This medium sheet can not only make body fluids transport into an absorption element quickly to enhance absorption performance by the absorption element but also prevent "back flow" phenomenon of body fluids from the absorption element once absorbed to always keep a dry state on the top sheet 30.

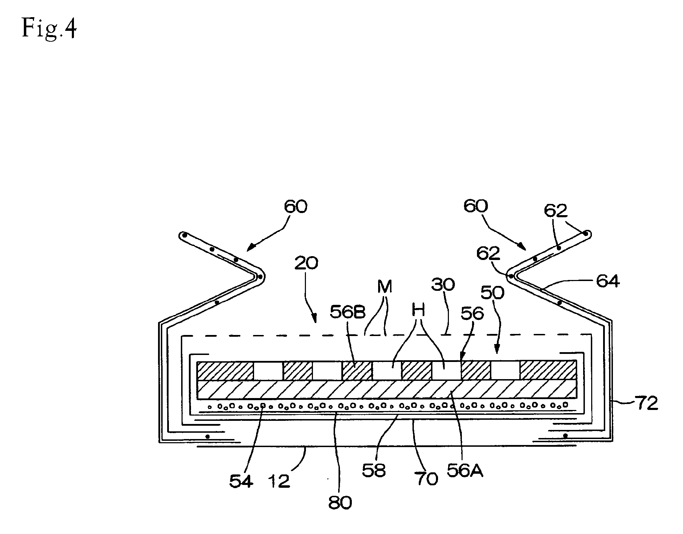

The medium sheet (second sheet) 40 is interposed between the top sheet 30 and wrapping sheet 58. A mode without disposing medium sheet (second sheet) 40 can be also used as shown in FIG. 4.

The medium sheet 40 shown in the figure is shorter than the width of an absorber 56 and disposed in the middle, may be disposed in the entire width. The length of medium sheet 40 in the longitudinal direction may be the same as the absorber 56 or in a range of shorter length centered in a region of receiving body fluids. A typical material of medium sheet 40 is non-woven fabric having excellent permeability to body fluid.

As the medium sheet 40, there can be exemplified the same material as the top sheet 30, spun lace, non-woven pulp, mixed sheet of pulp and rayon, point bond or crepe paper. In particular, air-through non-woven fabric and spun bond non-woven are preferred.

An elastic degree of medium sheet in the length direction of a product is preferably 0.05 to 0.75 g · cm²/cm to reduce or not to generate shingly uncomfortable feeling when touched from the surface side of a product. Herein, "elastic degree in the length direction of product" means a value obtained in such manner that a cut sample of 200 mm long and 200 mm wide is bent in DEF sensitivity of 20, in a range of curvature radius of 0.0 cm⁻¹ to 0.5 cm⁻¹, using a single bending tester (KES-FB2 manufactured by Kato Tech Co. Ltd.). This is the same as in a wrapping sheet.

### (Absorption element)

An absorption element 50 has an upper layer absorber 56B that hole M capable of passing loose stool is formed, and a lower layer absorber 56A provided thereunder. Also, a holding sheet 80 is provided between the absorbers 56B, 56A and the rear side region (downside part) a wrapping sheet 58.

In the thus constructed disposable diaper, loose stool excreted is passed through holes M of top sheet to move quickly to the upper layer absorber 56B side, further move quickly to the lower layer absorber side through the passage holes H of upper layer absorber 56B. In this process, part of liquid content is absorbed in the upper layer absorber 56B, most are reached to the lower layer absorber 56A to be absorbed and retained by the lower layer absorber 56A. Namely, both absorbers 56A and 56B have a function to absorb and retain excretion, characteristically, the lower layer absorber 56A mainly plays a function of retaining loose stool, and the upper layer absorber 56B plays a function of retaining a distance to maintain the lower layer absorber 56A retaining loose stool in a far place from skin.

### (Absorber)

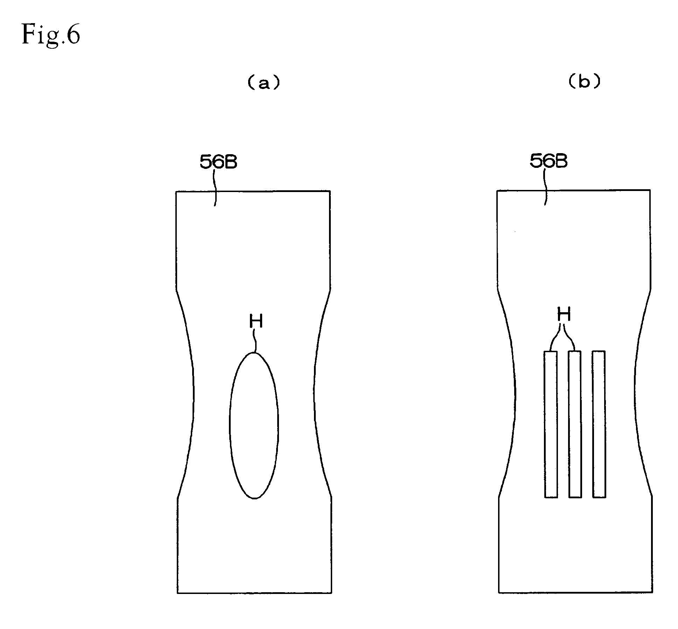

The open area ratio of passage hole H in the upper layer absorber 56B is preferably 5 to 40%, particularly preferably 10 to 20%. The shape and disposition are not particularly limited, as shown in FIG. 2, there can be suitably chosen a shape that a lot of small holes H of circle, triangle, square or lozenge are regularly or irregularly disposed, a shape that an excretion region are covered by relatively large holes H having an arbitral shape of ellipse etc. as shown FIG. 6 (a), and a shape that a plurality of holes H of long and narrow rectangle along the longitudinal direction are disposed in the width direction as shown FIG. 6(b). The diameter of a passage hole H is preferably 3 to 50 mm, particularly preferably 10 to 30 mm. Further, the number of passage holes H is preferably 1 to 20 per one absorber, particularly preferably 1 to 5. Such passage hole H can be formed by punching-out processing in production of upper layer absorber 56B.

At least one of the lower layer absorber 56A and the upper layer absorber 56B is formed by a tow opening fiber aggregate formed by opening tow. Namely, there can be adopted any structure that the lower layer absorber 56A is formed by a tow opening fiber aggregate, the upper layer absorber 56B is formed by a fiber laminate absorber formed by laminating a conventional short fiber such as pulp, reversely, the lower layer absorber 56A is formed by a fiber laminate absorber and the upper layer absorber 56B is formed by a tow opening fiber aggregate, and both lower layer absorber 56A and upper layer absorber 56B are formed by a tow opening fiber aggregate.

When the lower layer absorber 56A is formed by a fiber aggregate composed of tow, the lower layer absorber 56A is expanded as loose stool is absorbed to retain the solid content firmly. Then, the lower layer absorber 56A which retains loose stool is distanced from the top sheet by the upper layer absorber 56B. Thus, loose stool is accommodated and retained in a far position from skin as well as it is quickly kept a distance.

Also, when the upper layer absorber 56B is formed by a fiber aggregate composed of tow, the upper layer absorber 56B is excellent in shape retention and cushioning property due to fiber length, which can definitely maintain the lower layer absorber 56A which absorbs and retains loose stool in a far positions from skin.

### (Tow opening fiber aggregate)

A tow opening fiber aggregate is formed by opening a tow (fiber bundle) of bundle of filament essentially considered as a continuous fiber, namely, it is a filament aggregate. As a constituent fiber of tow, for example, there can be used polysaccharide and its derivatives such as cellulose, cellulose ester, chitin and chitosan, and synthetic polymers such as polyethylene, polypropylene, polyamide, polyester, polylactamide and polyvinyl acetate, cellulose ester and cellulose are particularly preferable.

As cellulose, there can be used cellulose derived from plant such as cotton, linter, wood pulp, and bacteria cellulose, regenerated cellulose like rayon may be used, regenerated cellulose may be a spun fiber.

As a preferable cellulose ester, for example, there can be used organic acid ester such as cellulose acetate, cellulose butyrate and cellulose propionate; mixed acid ester such as cellulose acetate propionate, cellulose acetate butyrate, cellulose acetate phthalate, and cellulose acetate nitrate; and cellulose ester derivatives such as polycaprolactone graft cellulose ester. These cellulose esters can be used alone or, in two or more kinds thereof. The viscosity-average degree of cellulose ester is, for example, 50 to 900, preferably about 200 to 800. The average degree of substitution in cellulose ester is, for example, about 1.5 to 3.0 (e.g. 2 to 3).

The average degree of cellulose ester can be, for example, 10 to 1000, preferably 50 to 900, further preferably about 200 to 800, the average degree of substitution in cellulose ester can be, for example, about 1 to 3, preferably 1 to 2.15, and further preferably about 1. 1 to 2.0. The average degree of substitution in cellulose ester can be selected from the points of enhancing biodegradation etc.

As a cellulose ester, organic acid ester (e.g. ester of organic acid having carbon atoms of about 2-4), cellulose acetate is particularly preferred. Acetylation degree of cellulose acetate is often about 43 to 62%, about 30 to 50% is particularly preferable due to good biodegradation. Particularly preferable cellulose ester is cellulose diacetate.

Constituent fiber of tow may contain, for example, heat stabilizer, pigment, oil, yield improving agent, whiteness improving agent and the like.

Fineness of constituent fiber of tow can be, for example, 1 to 16 deniers, preferably 1 to 10 deniers, and further preferably about 2 to 8 deniers. Constituent fiber of tow may be non-crimped fiber, preferably crimped fiber. Degree of crimp of crimped fiber can be, for example, 5 to 75 pieces per one inch, more preferable 10 to 50, and further preferable about 15 to 50. Also, there are many cases that crimped fiber uniformly crimped is used. When crimped fiber is used, a bulky and light-weight absorber can be produced and also highly integrated tow can be easily produced by intertwine of fibers. Cross section of constituent fiber of tow is not particularly limited, for example, may be circular, elliptical, non-circular (e.g. Y-shape, X-shape, I-shape, R-shape) or hollow shape. Constituent fiber of tow can be used in tow (fiber bundle) formed by bundling filaments of 3000 to 1000000 for example, preferably about 5000 to 1000000. It is preferable that fiber bundle is constructed by bundling continuous filaments of about 3000 to 1000000.

Tow is week in intertwine of fibers, thus mainly to maintain the shape, binders capable of adhesion or thermally bonding operation at contact parts of fibers can be used. The binder can employ ester based plasticizers such as triacetin, triethylene glycol diacetate, triethylene glycol dipropionate, dibutyl phthalate, dimethoxyethyl phthalate and triethyl citrate, in addition thereto, may employ various resin adhesives, particularly thermoplastic resins.

As a thermoplastic resin for binder, it is a resin exhibiting adhesion force resulting from melting and solidification, including water insoluble or water hardly soluble resin, and water soluble resin. Water insoluble or water hardly soluble resin and water soluble resin can be in concomitant use according to need.

As a water insoluble or water hardly soluble resin that can be used, for example, there are listed olefin based homopolymer or copolymer such as polyethylene, polypropylene, ethylene-propylene copolymer and ethylene-vinyl acetate copolymer, polyvinyl acetate, acrylic resins such as polymethyl methacrylate, methyl methacrylate-acrylate copolymer, and (meth)acrylic monomer-styrene monomer copolymer; polyvinyl chloride, vinylacetate-vinylchloride copolymer, polystyrene, styrene based polymer such as copolymer of styrene type monomer with (meth)acrylic type monomer; polyesters that may be modified; polyamide such as nylon 11, nylon 12, nylon 610 and nylon 612; rosin derivatives (e.g. rosin ester); hydrocarbon resins (e.g. terpene resin, dicyclopentadiene resin, petroleum resin); hydrogenated hydrocarbon resin. These thermoplastic resins can be used alone or, in two or more kinds thereof.

As a water soluble resin that can be used, there are listed various water soluble polymers, for example, polyvinyl alcohol, polyvinyl pyrrolidone, polyvinyl ether, vinyl based water soluble resins such as copolymers of vinyl monomer with copolymerizable monomer having a carboxyl group, sulfonic group or their salts, acrylic based water soluble resin, polyalkylene oxide, water soluble polyester and water soluble polyamide. These water soluble resins may be used alone or, in combination of two or more kinds thereof.

The thermoplastic resin may be added with various additives such as stabilizers like antioxidant and ultraviolet absorbing agent, filler, plasticizer, antiseptic and fungus proofing agent.

However, it should be avoided to use a binder component preventing super absorbent polymer particles from infiltration as far as possible. It is the best way not to use a binder component preventing super absorbent polymer particles from infiltration.

Tow can be produced in known methods, so it will not be described in detail. Tow bale of cellulose diacetate that can be preferably used for absorption element 50 is commercially available from Celanese Chemicals, Ltd. and Daicel Chemical Industries, Ltd. Tow bale of cellulose diacetate has a density of about 0.5 g/cm³, and has a total weight of 400-600 kg.

Tow is taken out from the bale, and opened in a wide belt to be a desired size and bulkiness. The width of opening tow is arbitral, for example, can be 100 to 2000 mm in width, preferably about 100 to 300 mm according to the width of absorber in a product. The basis weight and density of fiber aggregate can be adjusted by adjusting the degree of opening fibers.

The basis weight of tow opening fiber aggregate is 30 to 300 g/m², particularly preferably 30 to 90 g/m². The basis weight of fiber can be adjusted by selecting tow for original fabric or by the production conditions. Also, a tow opening fiber aggregate preferably has base density of 0.0075 g/cm³ or less in the thickness of 10mm, particularly preferably 0.0040 to 0.0070 g/cm³. When the fiber density is too high, there becomes few merit in using a fiber aggregate formed by opening tow, for example, weight saving and reduction of thickness become difficult.

Tow opening fiber aggregate is excellent in retention of body fluid when fiber density is low, on the other hand, it is excellent in diffusion of body fluid when fiber density is high. Accordingly, in the absorber composed of the tow opening fiber aggregate, it is preferable that the fiber density in both sides in the width direction is set to be lower than that of the middle part in the width direction. By setting such density difference, body fluids diffuses quickly in the middle in the width direction of absorber composed of tow opening fiber aggregate, retention of body fluid is improved in both sides in the width direction where quick diffusion is not required, which imparts the most preferable characteristics to each region. However, regardless of how much difference of fiber density in both sides and the middle part, the fiber density of both sides is preferably 10 to 100 g/m³, more preferably 20 to 70 g/m³, and particularly preferably 30 to 50 g/m³. When the fiber density of both sides is too low, bias in the width direction of absorber could occur, when the fiber density in both sides is too high, it could give a wearer uncomfortable feeling.

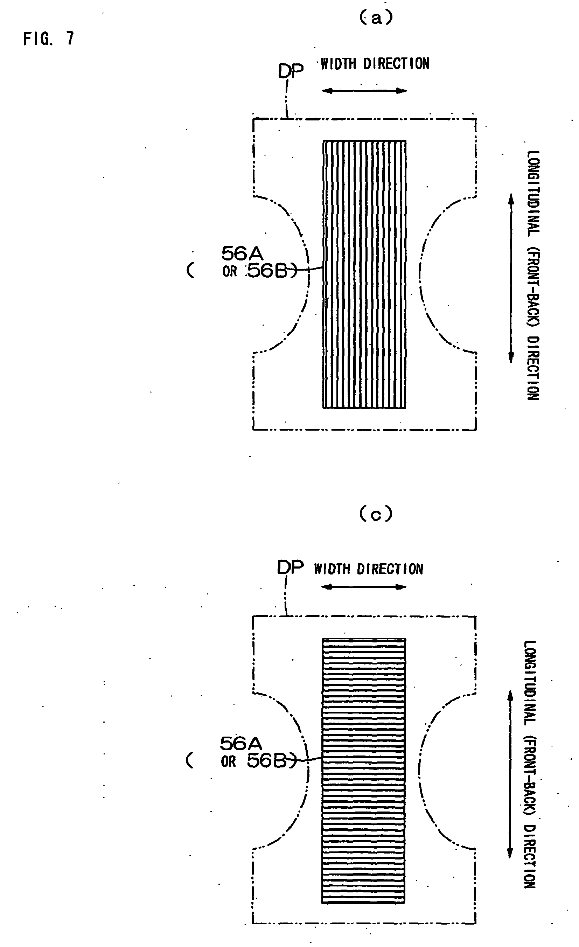

Tow opening fiber aggregate has a characteristic that body fluids diffuse easily along the continuous direction of fiber (flow direction), but hardly diffuse in the direction perpendicular to the continuous direction of fiber. Therefore, as shown in FIG. 7(a), an absorber 56A (or 56B) composed of tow opening fiber aggregate can be preferably formed so that the continuous direction of fiber of tow (expressed as many lines) is formed/disposed along the longitudinal direction of diaper DP (back and forth direction). When the continuous direction of fiber is set to the longitudinal direction of goods, fluid diffuses quickly in the longitudinal direction as well, which utilizes the whole face of absorber effectively. However, as shown in FIG. 7(b), it is possible to form and dispose the fiber continuous direction of tow along the width direction of diaper DP. At any rate, it is preferable to construct in such manner that fiber is to be continued along the lateral direction perpendicular to the thickness direction of absorber 56A (or 56B).

Additionally, as a method for opening tow, for example, there can be used a method that a tow is loaded to a plurality of opening rolls, in proceeding of tow, the width of tow is gradually enlarged, a method of opening tow by repeating strain (extension) and relaxation (construction) of tow, and a method of enlarging width/opening tow using compressed air.

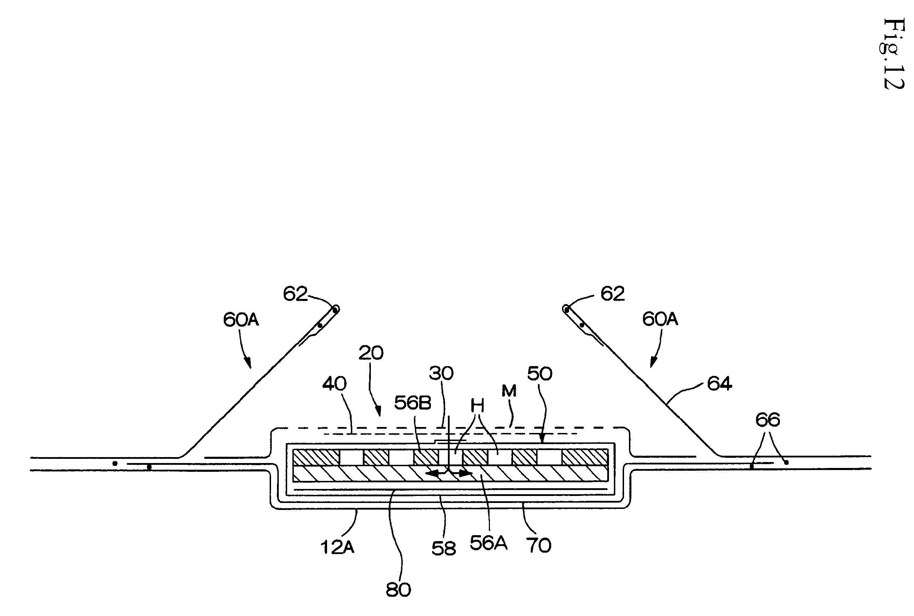

FIG. 12 shows an example of production facility for opening, tow 52 Y is taken out from a tow bale 52X sequentially, in the conveyor process, passed through an enlarging width /opening means 120 using compressed air, and through a opening part combined with a plurality of opening nip rolls 126A, 126B and 126C whose rotational speed is higher toward the downstream roll to widen and open , then passed through a binder adding box 140 to add a binder 140b (e.g. triacetin mist is filled in a box), thereby to give a fiber aggregate 52Z with desired width and density of tow.

### (Fiber laminate absorber)

On the other hand, a fiber laminate absorber can be produced by known methods that short fibers such as pulp are laminated and treated by intertwine with air and water. As the pulp fiber, there can be used chemical pulp obtained from wood, cellulose fiber such as dissolving pulp, and regenerated cellulose fiber such as rayon and acetate, softwood pulp with longer fiber length is preferably used than hardwood pulp in the points of performance and costs.

### (Super absorbent polymer)

Fiber aggregate 40 composed of tow can contain super absorbent polymer. As the super absorbent polymer particle, there are starch type, cellulosic and synthetic polymer types, there can be used carboxymethyl cellulose, polyacrylic acid and its salts, crosslinked polyacrylic acid salt, starch-acrylic acid graft copolymer, hydrolyzed product of starch-acrylic nitrile graft copolymer, crosslinked polyoxyethylene, crosslinked carboxymethyl cellulose, polyethylene oxide, partly crosslinked water-swelling polymer like plolyacryamide and isobutylene-maleic acid copolymer. It is possible to use a material that anti-blocking agent is added to suppress blocking due to moisture absorption of product.

Also, there are super absorbent polymers with various shapes such as particulate including powder, granule, pellet, sol, suspension, gel, film and non-woven, these are usable in the present invention, in particular, particulate shape is preferably used.
The particulate super absorbent polymer can preferably employ 20 to 850 µm in particle diameter. Specifically, super absorbent polymer particle having the following feature can be used.
Average particle diameter: about 350 µm
Distribution of particle size
850 µm on: o%
500 µm on: 12.2%
250 µm on: 75.7%
180 µm on: 8.8%
106 µm on: 2.4%
106 µm pass: 0.9%
primary particle diameter: 110-120 µm

As a super absorbent polymer, it can be basically used regardless of absorption performance, but the absorption amount of 50 g/g or more is preferable. Also, super absorbent polymer particle having an absorption speed of 45 seconds or less is preferable. When the absorption speed exceeds 45 seconds, so-called back flow that body fluids supplied into an absorption element flow back outside the absorption element tends to occur.

Also, super absorbent polymer particle with gel strength of 900 Pa or more can be preferably used. Thereby, in the case of bulky absorber obtained by using tow, sticky feeling after absorption of body fluid can be effectively suppressed.

The basis weight of super absorbent polymer can be suitably determined according to the amount required for applications of the absorption element. Thus, being not categorically described, for example, it can be 400 g/m² or less. When the basis weight of polymer is too low, absorption performance cannot be retained, whereas when it is too high, the effect is not only saturated but also the foregoing shingly uncomfortable feeling is given due to excess of super absorbent polymer particles.

As a method for containing super absorbent polymer in a tow opening fiber aggregate, there can be adopted a method of scattering or projecting particulate super absorbent polymer from outside, a method of polymerizing after monomer capable of becoming super absorbent polymer is immersed into a fiber aggregate composed of tow, and a method of crosslinking treatment after a fiber aggregate composed of tow is coated with uncrosslinked super absorbent polymer gel. For fiber laminate absorber, super absorbent polymer can be also contained in the absorber by the conventional methods such as air-lay method and polymerization method.

Super absorbent polymer may be retained in a fiber aggregate, on a surface of fiber aggregate, or both. Moreover, super absorbent polymer may be partly retained on a surface of fiber aggregate, may be retained entirely in a fiber aggregate. Also, super absorbent polymer can be disposed in a layer on at least one side of front face and back surface of fiber aggregate. In particular, a layer of super absorbent polymer is preferably disposed on the back surface side of fibber aggregate. In these cases, super absorbent polymer can be bonded to a sheet wrapping a fiber aggregate with adhesives. As the adhesive in this case, the foregoing thermoplastic resins can be used.

It is desirable for a fiber aggregate that super absorbent polymer particles (SAP particles) are dispersed essentially in the whole thickness direction at least in a region receiving body fluids. In the case where there is no SAP particle in the upper, under and middle parts of fiber aggregate, there can be hardly said that it is "dispersed wholly in the thickness direction". Therefore, "dispersed wholly in the thickness direction" means a mode where it is "uniformly" dispersed wholly in the thickness direction for a fiber aggregate, in addition thereto, includes a mode where "it is biased" in upper, under and middle parts, still dispersed in each part of upper, under and middle parts. Also, there is no exclusion of modes where parts of SAP particles do not enter into a fiber aggregate, but stay on the surface; parts of SAP particles pass through a fiber aggregate and are present on the wrapping sheet 58; and they are present on the holding sheet 80 as shown in FIG. 4. Additionally, in the case where gel blocking is not concerned, they may be biased only in the upper part or only in the middle part, in the case where back flow is not concerned, they may be biased only in the middle part or only in the under part.

According to need, super absorbent polymer particles can be adjusted in scattering density or application amount in the plane direction of a product. For example, the application amount in an excretion region of body fluid can be increased more than other region. In the case of considering difference of man and woman, the scattering density (amount) can be increased in the front side for man, and the scattering density (amount) can be increased in the middle for woman. Also, a region with no presence of polymer can be disposed locally, like a spot for example, in the plane direction of product.

Namely, regarding all directions of width direction, longitudinal-direction and thickness direction of product, in addition to a mode that the amount of super absorbent polymer particle is uniform, regarding at least one direction of width direction, longitudinal direction and thickness direction of product, a mode that the amount of super absorbent polymer particles (hereinafter called dispersion density) in each part is large or small can be adopted.

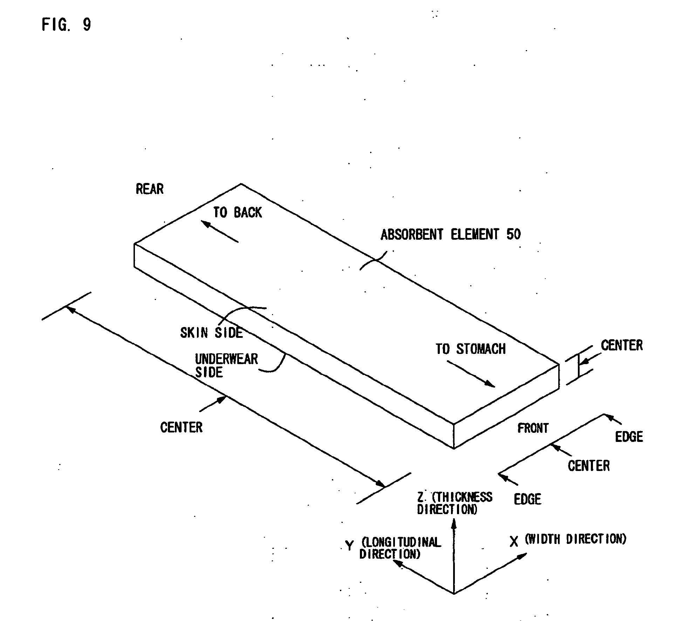

Summary of a mode "dispersion density of super absorbent polymer particles being large and small" is as follows. As shown in FIG. 9, in regard to absorption element 50, when the width direction of a product is X, the longitudinal direction is Y, and the thickness direction is Z, as shown in Table 1, the case where dispersion density of super absorbent polymer particles in one region is enlarged (enhanced) more than that in other region is defined as "bias", the case where dispersion density of super absorbent polymer particles is the same is defined as "uniform", specific effect of each mode is shown in Table 2 through Table 4. Needless to say, a combination of each condition can be used.

[Table 1]

[Table 2]

[Table 3]

[Table 4]

The ratio of super absorbent polymer particle and fiber aggregate determines absorption characteristics. As the weight ratio in a plane area of 5 cm×5 cm in a region directly receiving body fluid, super absorbent polymer particle/filament by weight is desirably 1 to 14, particularly 3 to 9.

### (Size and weight of absorber)

Lower layer absorber 56A and upper layer absorber 56B preferably have a whole plan project area of 250 cm² or more. Also, the thickness of lower layer absorber 56A and upper layer absorber 56B can be suitably determined, it is preferable to construct lower layer absorber 56A and upper layer absorber 56B in such way that a total thickness (summation of thickness of lower layer absorber 56A and length of upper layer absorber 56B) is 3 to 20 mm, particularly 5 to 15 mm when absorptive goods is taken out from package, allowed to stand in room at a temperature of 23 degree and humidity of 50% for one day. When the size of lower layer absorber 56A and upper layer absorber 56B is in this range, it is very advantageous for improvement on resilience without increasing weight, thickness and costs. The thickness of lower layer absorber 56A and upper layer absorber 56B may be different, particularly, it is preferable to keep a sufficient amount of accommodation for loose stool by setting the lower layer absorber 56A thicker than the upper layer absorber 56B. Also, it is preferable to construct in such way that the weight of absorber is set to be 25 g or less, particularly 10 to 20 g.

### (Compression characteristics of absorber)

Compression resilience RC of lower layer absorber 56A and upper layer absorber 56B is preferably 40 to 60%, particularly preferably 50 to 60%. Thereby, an absorber itself can exhibit sufficient resilience.

Further, when compression energy WC of lower layer absorber 56A and upper layer absorber 56B is 4.0 to 10.0 gf · cm/cm², it is possible to be compressed compactly in the same level as conventional or more in packing.

These compression characteristics can be adjusted through the adjustment of fiber density of fiber aggregate by opening etc., selection of fiber material, selection of the kind of binder like plasticizer and the adjustment of degree of treatment thereof, or combinations thereof.

Herein, compression energy (WC) is an amount of energy consumption when a test piece cut in length of 200 mm and width of 50 mm is pushed at the center part up to 50 g (thickness of this case is used in Examples).

This compression energy can be measured by a handy compression tester (KES-G5 manufactured by Kato Tech Co. , Ltd.). The conditions in using this tester are: SENS: 2, the kind of pressure gauge: 1kg, SPEED RANGE: STD, DEF sensitivity: 20, pressure area: 2 cm², input intervals: 0.1 (standard), STROKE SET: 5.0, upper limit load: 50 gf/cm²).

Moreover, compression resilience (RC) is a parameter expressing resilience when fiber is compressed. Therefore, compression resilience becomes large when resilience is good. This compression resilience can be measured by a handy compression tester (KES-G5 manufactured by Kato Tech Co,. Ltd.). The conditions in using this tester are the same as the above compression energy.

### (Wrapping sheet)

As wrapping sheet 58, there can be used tissue paper, particularly crepe paper, non-woven fabric, poly-laminated non-woven fabric, and a sheet with small open holes. In the case of containing super absorbent polymer particles, a sheet that super absorbent polymer particles cannot be slipped out is desirable. Also, a sheet having absorption such as crepe paper is preferable. In the case of using non-woven fabric instead of crepe paper, hydrophilic non-woven fabric SMMS (spun bond/melt blown/melt blown/spun bond) is particularly preferable, the material can employ polypropylene, polyethylene/polypropylene, or the like. The basis weight is desirably 8 to 20 g/m², particularly desirably 10 to 15 g/m².

As shown in FIG. 3, it is a mode that this wrapping sheet 58 wraps a layer of fiber aggregate and super absorbent polymer particles 54 entirely, in addition thereto, for example as shown in FIG. 4, only the back surface and side surface of the layer may be wrapped. Also, not shown in a figure, there may be a mode that only the upper surface and side surface of absorbers 56A and 56B are wrapped with crepe paper or non-woven fabric, the under surface is wrapped with a body fluid impermeable sheet such as polyethylene and a mode that only the upper surface of absorber 56 is wrapped with crepe paper or non-woven fabric, the side surface and under surface are wrapped with a body fluid impermeable sheet such as polyethylene (these respective materials become respective constituents of wrapping sheet). If necessary, there may be a case that a layer of fiber aggregate and super absorbent polymer particles 54 is tucked up and down with two sheets, and a case that it is disposed only in the under surface or the upper surface, but which is not a desirable mode because movement of super absorbent polymer particles can be hardly prevented.

### (Holding sheet)

Super absorbent polymer particles 54 can be present by scattering them between a holding sheet 80 and absorbers 56A, 56B. There are instances that super absorbent polymer particles 54 are passed through a fiber aggregate in scattering/projecting them on a fiber aggregate or in the following steps, or in distribution processes to use by consumers. The concavity and convexity of super absorbent polymer particles passed trough a fiber aggregate give a shingly uncomfortable feeling when touched by hand in use by a consumer. Therefore, holding sheet 80 having upholding characteristic of absorptive polymer is disposed between the absorbers 56A, 56B and wrapping sheet 58. This holding sheet 80 reinforces elastic property that is not sufficient by wrapping sheet 58 like tissue paper (crepe paper) alone, and reduces or prevents uncomfortable feeling when touched by hand in use by a consumer.

Additionally, FIG. 4 shows schematically a case where super absorbent polymer particles passed out through a fiber aggregate are gathered on the holding sheet 80 in the steps from production to use by consumers when super absorbent polymer particles are provided down below the lower layer absorber 56A, or contained in the lower layer absorber 56A.

Material of holding sheet 80 is not particularly limited, any one with retaining performance of absorptive polymer is sufficient. Specifically, for example, there can be exemplified non-woven fabric, crimped pulp, low-absorptive cotton fibers such as non-degreased cotton fiber, degreased cotton fiber, rayon fiber treated with water repellent agent or hydrophobic agent; polyethylene fiber, polyester fiber, acrylic fiber, polypropylene fiber, silk, cotton, linen, nylon, polyurethane and acetate fibers.

In the case of non-woven as holding sheet 80, the holding sheet 80 is preferably a non-woven fabric with the compression energy based on KES test is 0.01 to 10.00 gf cm/cm², preferably 0.01 to 1.00 gf cm/cm² and the compression resilience of 10 to 100%, preferably 70 to 100%. Also, elastic degree of holding sheet 80 in the back and forth direction of a product is 0.05 to 0.75 g **·** cm²/cm to reduce or prevent a shingly uncomfortable feeling resulted from super absorbent polymer. Herein "elastic degree in the back and forth direction of a product" means a value obtained when a sample cut to a length of 200 mm and width of 200 mm is bent using a single bending tester (KES-FB2 manufactured by Kato Tech Co,. Ltd.) with DEF sensitivity of 20 and curvature range of 0.0 cm⁻¹ to 0.5 cm⁻¹.

As mentioned above, the reason for providing a holding sheet 80 is to retain absorptive polymer fallen off (passed out) downward from a fibber aggregate for example. Thus, the super absorbent polymer particles passed out touch a wearer through wrapping sheet 58 and holding sheet 80, so that there is no fear to pass on a shingly unconformable feeling to a wearer. Particularly, in the case of non-woven fabric with the above compression energy and compression resilience, function as a holding sheet is sufficiently exhibited.

Also, the absorptive polymer passed out are retained by the holding sheet 80, do not move onto the wrapping sheet 58, so that there is no fear to generate deviation of absorption performance. In particular, to prevent super absorbent polymer particles from moving onto the holding sheet 80, a hot melt adhesive with adhesion can be applied to the holding sheet 80 beforehand. Also, the upper surface (face facing use-face) of holding sheet 80 may be processed into a rough face to prevent super absorbent polymer particles from moving onto the holding sheet 80. As means for making surface rough or for carding for this purpose, there can be listed the use of non-net face which is a reverse face touching a net in production of non-woven fabric, a marble treatment, processing by needle punch and brushing treatment

The holding sheet 80 may be disposed only down below the absorber 56 as shown in FIG. 3, or may be extended by windup passing through the side of absorbers 56A and 56B to the upper surface of absorbers 56A, 56B. A plurality of holding sheets 80 can be used by piling them up.

The above example is an example that holding sheet 80 is disposed between the absorbers 56A, 56B and the back surface region of wrapping sheet 58, the holding sheet 80 may be in back surface side of wrapping sheet 58 (the mode is not shown), or without purposely disposing the holding sheet 80, wrapping sheet 58 itself may be functionalized as a holding sheet, in brief, holding sheet 80 is disposed in the back surface side of absorbers 56A, 56B, which reduces and prevents a shingly uncomfortable feeling when touched from the back surface side of a product.

### (Body fluid impermeable sheet)

Body fluid impermeable sheet 70 means a sheet disposed simply in the back surface side of absorbers 56A, 56B, a sheet for absorbers 56A and 56B to be interposed between the sheet and top sheet 30 in the present embodiment. Thus, the material of the body fluid impermeable sheet is not particularly limited. Specifically, for example, there can be exemplified olefin based resins such as polyethylene and polypropylene, laminate non-woven fabrics laminated with non-woven and polyethylene sheet etc., non-woven fabric that a water proof film is interposed therein to essentially keep impermeability (in this case, body fluid impermeable sheet is composed of water proof film and non-woven). Needless to say, in addition thereto, a material having liquid impermeability and moisture permeability preferably used recently from the prevention of stuffy feeling can be exemplified. As a sheet of material having liquid impermeability and moisture permeability, for example, there can be a microporous sheet obtained in such manner that inorganic fillers are kneaded in a olefin based resin such as polyethylene and polypropylene to form a sheet, followed by stretching uniaxially or biaxially.

The body fluid impermeable sheet 70 is extended to use-surface in a so-called forehead wrapping (not shown), thereby side leakage of body fluid can be prevented, in the embodiment, the side leakage is prevented by a second body-fluid impermeable sheet 72 disposed between two-hold barrier sheet 64 forming a barrier cuff 60. Based on this mode, since the second body-fluid impermeable sheet 72 is extended to the standing of barrier cuff 60, there are merits that side leakage of body-liquid diffused crosswise being run through top sheet 30 and loose stool between barrier cuffs 60 can be prevented.

### (Barrier cuff)

Barrier cuffs 60 provided in both sides of a product are provided in order to stop urine and loose stool moving crosswise being run through top sheet 30 and to prevent side leakage, but they are an augmentative element.

Barrier cuff 60 shown in the figure is formed by two-holding the barrier sheet, it is formed by covering the folded part of top sheet 30 downwards from the back surface side of absorbers 56A, 56B and protruding to surface side. In order to prevent urine moving crosswise in running onto the top sheet 30, in particular, the side part of body-fluid impermeable sheet 70 is inserted between two-hold non-woven fabric, which extends in the partway of barrier cuff 60 protruding to the surface side

Also, the shape of barrier cuff 60 itself can be suitably designed, in the example shown in the figure, an elastic stretch member such as rubber thread 62 is fixed under tension in the end and the middle part of protruding part of barrier cuff 60, barrier cuff 60 stands by the contractive force in a use state. The rubber thread 62 of middle part is located nearer to the center than the rubber threads 62 in the end, fixed in the back and forth ends of top sheet 30, thus, as shown in FIG. 3, the base part of barrier cuff 60 stands at a slant towards the center and becomes a state that the end from the middle part stands outwards at a slant.

Material of barrier sheet may have a permeable property to body fluid or may have a impermeable property to body fluid, and the kind is not particularly limited. For example, the same material exemplified as top sheet 30 and body fluid non-permeable sheet 70 can be employed. However, non-woven is preferable from the points of skin touch and prevention of skin fit due to friction, bulky non-woven fabric such as air through non-woven is more preferable.

Also, according to functions emphasized, water repellant non-woven fabric or hydrophilic non-woven respectively can be used alone or in combination thereof. For example, in the case of emphasizing prevention of body liquid permeation or improvement of skin touch, water repellant non-woven fabric is, for example, preferably a water repellent-treated non-woven coated with silicone based, paraffin based, alkyl chromic chloride based water repellants. On the other hand, in the case of emphasizing absorption of body liquid, hydrophilic non-woven fabrics can be used such as non-woven fabric made of hydrophilic natural fiber, synthetic fiber and regenerated fiber for example, and non-woven fabric of non-hydrophilic fiber to which hydrophilic treatment is given with hydrophilic agents.

### (Elastic stretch member)

As an elastic stretch member, its kind is not particularly limited as long as it has stretchablility. For example, stretch hot melt, stretch film, rubber thread and flat rubber can be exemplified. Also, as the material, for example, there can be exemplified styrene based, olefin based, urethane based and ester based rubbers; and foams of polyurethane, polyethylene, polystyrene, styrene-butadiene, silicone and polyester.

### (Embossing)

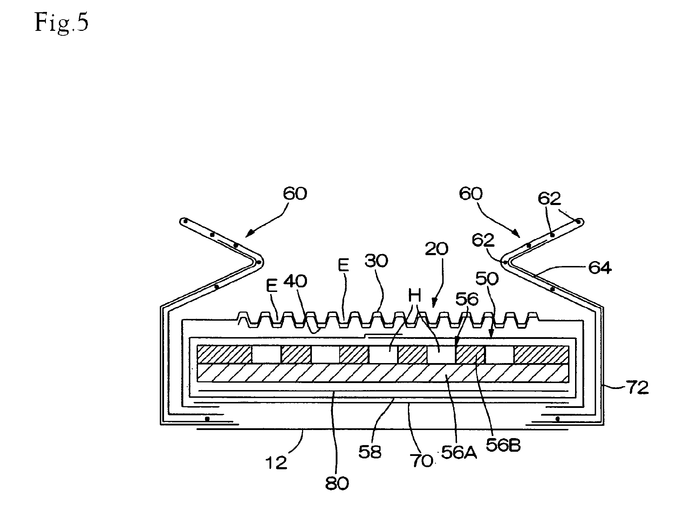

Concavity E may be formed by embossing in the thickness direction of top sheet 30 from the surface side. In this case, concavity E may be formed by embossing only top sheet 30, in addition thereto, concavity E may be formed by embossing both top sheet 30 and medium sheet 40 as shown in FIG. 5, and concavity may be formed by embossing to reach a part or almost entire of absorbers 56A, 56B in the thickness direction from the surface of top sheet 30 (not shown in a figure) . In order to form concavity E by embossing both top sheet 30 and medium sheet 40, the medium sheet 40 is preferably in a range of 8-40 g/m² in basis weight and 0.2-1.5 mm in thickness, the top sheet 30 is preferably in a range of 15-80 g/m² in basis weight and 0.2-3.5 mm in thickness, from the points that sufficient embossing can be done in the conditions without disturbing liquid permeability.

Also, without forming concavity on top sheet 30, concavity may be formed by embossing on only medium sheet 40, further, without forming concavity on top sheet 30 and medium sheet 40, concavity may be formed by embossing only absorbers 56A, 56B, without forming concavity on top sheet 30, medium sheet 40 and wrapping sheet 58, concavity may be formed by embossing on only absorbers 56A, 56B.

Concavity E has an effect to induce and diffuse body fluids in its extending direction. Thus, when concavity E is constructed essentially continuously in a channel, including the case where a plurality of concavities are rowed at intervals to form a channel, body fluids diffuse through concave E of the surface side layer before reaching absorbers 56A and 56B, which can utilize a wider part of absorbers 56A and 56B for absorption. Therefore, absorption capacity of a product as a whole increases, which provides absorption goods that leakage from the side and back flow due to lack of absorption capacity hardly occur.

On the other hand, it is preferable to form concavity by embossing on a fiber aggregate because rigidity can be increased. Being not shown in a figure, to increase rigidity of absorption element 50, it is also a preferable mode to form concavity by embossing from the back surface side of absorber 56A, 56B (opposite side to top sheet 30 side) in the thickness direction. To form concavity on this back surface side, an integrated embossing can be done so as to reach absorber 56A, 56B from the back surface side of holding sheet 80, wrapping sheet 58, body liquid non-permeable sheet 70 or external sheet 12. Also, such concavity of the back surface side is preferably formed together with concavity E of surface side, concavity of only back surface side can be formed without forming concavity E of surface side. In the case of forming concavity on both surface and back face sides, shape of concavity may be common in both surface and back face, or may be different each other in surface and back face.

Concavity by embossing has an effect to induce and diffuse body fluids in its extending direction. Also, it has an effect to increase rigidity. Thus, it is preferable to determine the shape of concavity by embossing in consideration of these effects. For example, in addition to the concavity being essentially continuously in a channel, including the case where a plurality of concavities are rowed at intervals to form a channel, a plurality of concavities may disposed in spots at intervals. Also, as a plane pattern, there can be adopted various shapes that channel or punctiform concavity is aligned in the longitudinal direction, in the width direction of product or in lattice combined therewith, zigzag configuration reciprocating in the width direction, or irregularly disposed pattern. Further, shapes such as pin-shape, Mt. Fuji-shape and accordion-shape can be suitably adopted.

### (Others)

Additionally, not shown in a figure, each constitutional member of absorptive body 20 is fixed each other with hot melt adhesive etc. In the case of applying adhesives, a part to be applied and a part not to be applied in bond area can be intentionally provided. Also, additives can be applied by curtain, spiral, slot, controlled seam (Omega-shaped) and bead applications.

### (Example of tape-type disposable diaper)

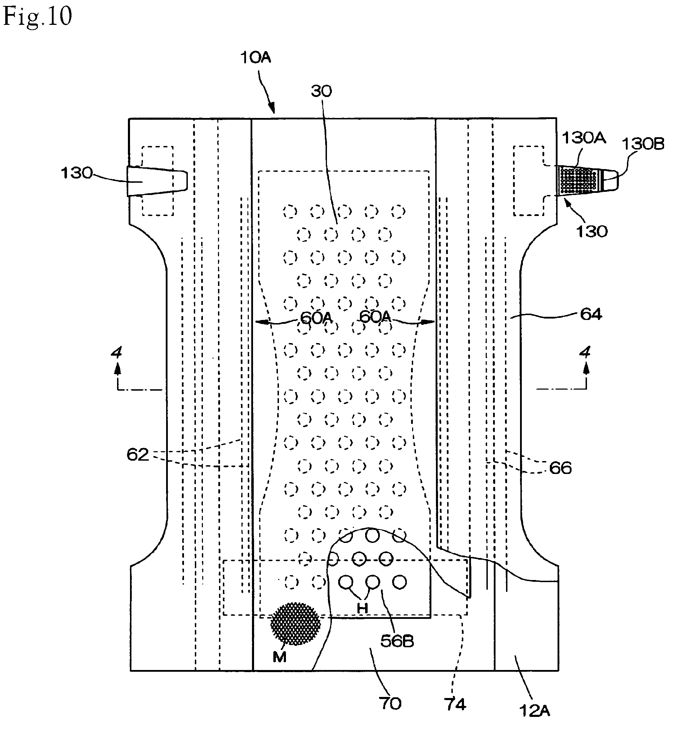

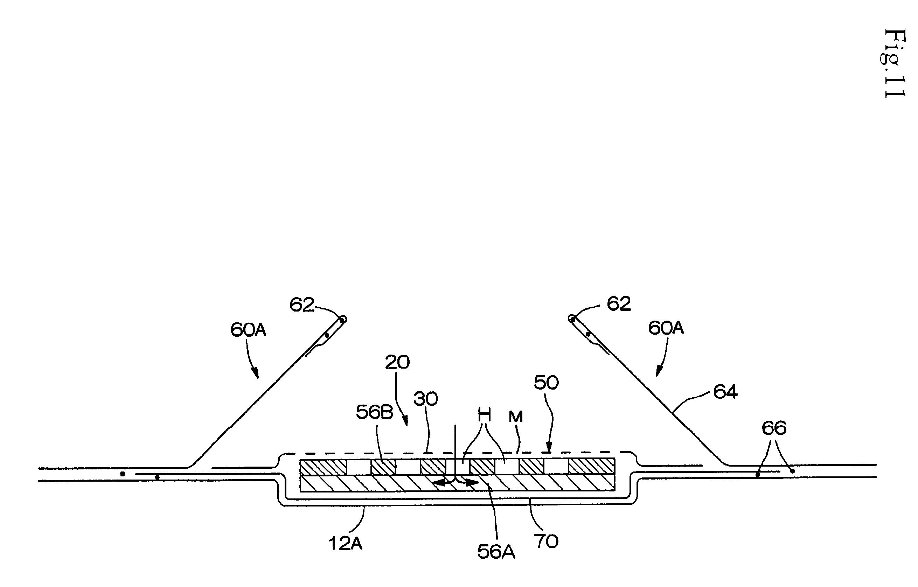

Now, FIG. 10 and FIG. 11 show an example of tape-type disposable diaper. FIG. 11 is a sectional view along 4-4 line in FIG.10, and absorptive body 20 is drawn somewhat in an exaggerated form.

Tape-type disposable diaper 10A has a fastening piece provided in both ends of diaper back surface side, has a hook element on a fixing surface of this fastening piece and non-woven laminate as a back sheet composing the back face of diaper, in wearing a diaper, the diaper is devised so that the hook element of fastening piece can be caught in any place on the surface of back sheet.

Absorptive body 20 is constructed with an upper layer absorber 56B that passage holes H capable of passing loose stool are formed and a lower layer absorber 56A between top sheet 50 and body liquid impermeable sheet 70. The body liquid impermeable sheet 70 is a rectangle wider than absorbers 56A, 56B, provided with a back sheet 12A of non-woven fabric in a sand clock shape outward. The top sheet 30 is a rectangle wider than absorbers 56A, 56B, extended outward somewhat over the side edges of absorbers 56A and 56B, bonded to the body liquid impermeable sheet 70 with hot melt adhesive etc.

As shown in FIG. 12, in tape-type disposal diaper, a medium sheet 40 can be interposed between the top sheet 30 and absorbers 56A, 56B. Also, entire or part of absorbers 56A and 56B can be wrapped with a wrapping sheet 58. Further, a holding sheet 80 can be disposed in the back surface side of absorber 56A, 56B.

Barrier cuff 60A protruding to use-surface side is formed on both sides of diaper, and this barrier cuff 60A is composed of a barrier sheet 64 of essentially continuous non-woven fabric in the width direction and rubber thread 62 as an elastic stretch member for leg periphery consisting of a piece or a plurality of pieces of rubber thread for example. A fastening piece in hook-and-loop fastener is denoted as 130.

Inner face of barrier sheet 64 has a starting edge for adhesion in a place distanced from the side edge of top sheet 30, from this starting edge for adhesion over the extended edge of body fluid non-permeable sheet 70, the outward part in the width direction is bonded with hot melt adhesive etc. The outer face of barrier sheet 64 is bonded in its under surface to the back sheet 12A with hot melt adhesive etc. Further, elastic stretch member for gasket cuff, for example, rubber thread 66 is provided.

The starting edge for adhesion to the body fluid impermeable sheet 70 in the inner face of barrier sheet 64 forms a standing edge of barrier cuff 60A. The inner side of this standing edge around a leg is a free part not fixed with product body, and this free part stands by contraction force of rubber thread 62.

In the present example, using hook-and-loop fastener as fastening piece 130, it can be mechanically fixed to back sheet 12A. Thus, a so-called target tape can be omitted, and fixing position can be arbitrarily chosen by the fastening piece 130.

Regarding fastening piece 130, base of fastening base material such as plastic, poly-laminated non-woven and paper is bonded to back sheet 12A with adhesives for example, it has a hook element 130A in the edge side. The hook element 130A is bonded to the fastening base material with adhesives. The hook element 130A has a lot of hooks in its outer face. It has a temporary adhesive part 130B at the edge of hook element 130A. In a final stage of fabricating a product, the temporary adhesive part 130B is bonded to a barrier sheet 64 to prevent peeling off the edge side of fastening 130. On occasion of use, it is peeled off against the adhesion force, the edge of fastening piece 130 is brought to front body. In the tip side over the temporary adhesive part 130B, fastening base material is exposed as a tab part.

In the opening side of front body, a target print sheet 74 as a design sheet is provided in the inner side of back sheet 12A, on which a target print designed to show an indication of place for fixing the hook element 130A of fastening piece 130 is conducted, which can be recognized from outside through back sheet 12A.

In wearing a diaper, the diaper is worn around a body in a ship shape and contraction force of rubber thread 62 is exerted, so that barrier cuff 60A stands around legs due to contraction force of rubber thread 62.

A space surrounded by standing part forms a space for blocking urine or loose stool. When urine is discharged in the space, the urine is passed through top sheet 30 and absorbed in absorbers 56A and 56B, and climbing of solid part in loose stool is prevented by the barrier of standing part of barrier cuff 60A. In case when urine is leaked crossing over the standing distal edge of standing part, side leakage is prevented by a stopping function of plane touching part.

In the present mode, barrier sheet 64 forming each of standing cuffs is preferably not permeable but essentially impermeable or may be semi-permeable for liquid. Also, barrier sheet 64 may be treated with silicone to provide a liquid repellant property. At any rate, the barrier sheet 64 and back sheet 12A each are air-permeable and, the barrier sheet 64 and back sheet 12A each preferably are a sheet with a water resisting pressure of 100 mm H₂O or more. By this way, side part in the width direction of product becomes air permeable, and stuffy state of wearer can be prevented.

Regarding other points, for example, for materials used in each part, the explanations are skipped daringly because they are the same as in the foregoing underpants-type disposable diaper.

### [Industrial Applicability]

The present invention is described in detail with reference to an example of so-called tape-type disposable diaper that both edges of right and left of the back surface side are put together with belly side in use (wearing), the present invention can be applied to an underpants-type disposable diaper that both edges of right and left of the back surface side and both edges of right and left of the belly side are fixed beforehand, and to a disposable diaper etc. of other mode.

### [Brief Description of Drawings]

FIG. 1 is a perspective view of underpants-type diaper.
FIG. 2 is a plan view of underpants-type diaper in a development state.
FIG. 3 is a sectional view along 3-3 line of FIG. 2.
FIG. 4 is a sectional view showing another example.
FIG. 5 is a sectional view showing a further example.
FIG. 6 is a plan view showing an absorber.
FIG. 7 is a plan view showing an absorber.

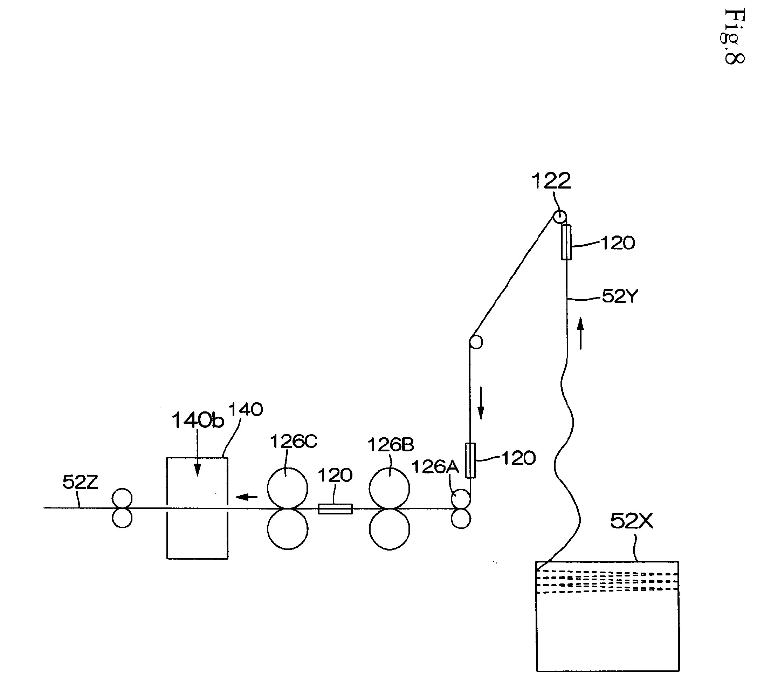

FIG. 8 is a brief overview showing a facility for opening tow.
FIG. 9 is a brief overview explaining the directions of absorption element.
FIG. 10 is a plan view of tape-type diaper in a development state.
FIG. 11 is a sectional view along 4-4 line of FIG. 10.
FIG. 12 is a sectional view showing another example. [Description of Reference Numbers]

10. Underpants-type disposable diaper
10A. Tape-type disposable diaper
12. External sheet
12A. Back sheet
20. Absorptive body
30. Top sheet
40. Medium sheet
50. Absorption element
52. Filament
52X. Bale
52Y. Tow
52Z. Fiber aggregate
54. Super absorbent polymer particle
56. Absorber
58. Wrapping sheet
60, 60A. Barrier cuff
64. Barrier sheet
70. Body liquid impermeable sheet
72. Second body liquid impermeable sheet
80. Holding sheet
130. Fastening piece
E. Concavity
Z. Scattering zone of super absorbent polymer particle

**[Table 1]**

| | | | | |
|---|---|---|---|---|
| X(width direction) | Near to center | Uniform | Near to edge | |
| Y(longitudinal direction) | Near to stomach | Near to center | Uniform | Near to back |
| Z(thickness direction) | Near to center | Uniform | Skin side | Underwear side |

**[Table 2]**

| In respect of X direction, when "near to center", SAP can be provided efficiently at necessary sites, thus enabling manufacturing at low costs, as well as SAP loss at the time of manufacturing is less. | | | | |
|---|---|---|---|---|
| X | Y | Z | Characteristic advantages | |
| Near to center | Near to stomach | Near to center | Advantageous for boys, less SAP discomfort. | (1) |
| | | Uniform | Advantageous for boys, good balance of absorption performance. | (2) |
| | | Skin side | Advantageous for boys, almost no back flow. | (3) |
| | | Underwear side | Advantageous for boys, high absorption speed. | (4) |
| | Near to center | Near to center | Advantageous for girls, less SAP discomfort. | (5) |
| | | Uniform | Advantageous for girls, good balance of absorption performance. | (6) |
| | | Skin side | Advantageous for girls, almost no back flow. | (7) |
| | | Underwear side | Advantageous for girls, high absorption speed. | (8) |
| | Uniform | Near to center | Advantageous for both boys and girls, less SAP discomfort. | (9) |
| | | Uniform | Advantageous for both boys and girls, good balance of absorption performance. | (10) |
| | | Skin side | Advantageous for both boys and girls, almost no back flow. | (11) |
| | | Underwear side | Advantageous for both boys and girls, high absorption speed. | (12) |
| | Near to back | Near to center | Advantageous for few month-old-babies e.g., newborn babies, less SAP discomfort. | (13) |
| | | Uniform | Advantageous for few month-old-babies e.g., newborn babies, good balance of absorption performance. | (14) |
| | | Skin side | Advantageous for few month-old-babies e.g., newborn babies, almost no back flow. | (15) |
| | | Underwear side | Advantageous for few month-old-babies e.g., newborn babies, high absorption speed. | (16) |

**[Table 3]**

| In respect of X direction, when "uniform", discomfort with SAP is reduced, and less SAP movement in use is achieved. | | | | |
|---|---|---|---|---|
| X | Y | Z | Characteristic advantages | |
| Uniform | Near to stomach | Near to center | Advantageous for boys, less SAP discomfort. | (17) |
| | | Uniform | Advantageous for boys, good balance of absorption performance. | (18) |
| | | Skin side | Advantageous for boys, almost no back flow. | (19) |
| | | Underwear side | Advantageous for boys, high absorption speed. | (20) |
| | Near to center | Near to center | Advantageous for girls, less SAP discomfort. | (21) |
| | | Uniform | Advantageous for girls, good balance of absorption performance. | (22) |
| | | Skin side | Advantageous for girls, almost no back flow. | (23) |
| | | Underwear side | Advantageous for girls, high absorption speed. | (24) |
| | Uniform | Near to center | Advantageous for both boys and girls, less SAP discomfort. | (25) |
| | | Uniform | - | (26) |
| | | Skin side | Advantageous for both boys and girls, almost no back flow. | (27) |
| | | Underwear side | - | (28) |
| | Near to back | Near to center | Advantageous for few month-old-babies e.g., newborn babies, less SAP discomfort. | (29) |
| | | Uniform | Advantageous for few month-old-babies e.g., newborn babies, good balance of absorption performance. | (30) |
| | | Skin side | Advantageous for few month-old-babies e.g., newborn babies, almost no back flow. | (31) |
| | | Underwear side | Advantageous for few month-old-babies e.g., newborn babies, high absorption speed. | (32) |

**[Table 4]**

| In respect of X direction, when "near to edge", it is advantageous for the reduction in leakage around legs or from sides. In particular, it is superior for use with an inner pad. | | | | |
|---|---|---|---|---|
| X | Y | Z | Characteristic advantages | |
| Near to edge | Near to stomach | Near to center | Advantageous for boys, less SAP discomfort. | (33) |
| | | Uniform | Advantageous for boys, good balance of absorption performance. | (34) |
| | | Skin side | Advantageous for boys, almost no back flow. | (35) |
| | | Underwear side | Advantageous for boys, high absorption speed. | (36) |
| | Near to center | Near to center | Advantageous for girls, less SAP discomfort. | (37) |
| | | Uniform | Advantageous for girls, good balance of absorption performance. | (38) |
| | | Skin side | Advantageous for girls, almost no back flow. | (39) |
| | | Underwear side | Advantageous for girls, high absorption speed. | (40) |
| | Uniform | Near to center | Advantageous for both boys and girls, less SAP discomfort. | (41) |
| | | Uniform | Advantageous for both boys and girls, good balance of absorption performance. | (42) |
| | | Skin side. | Advantageous for both boys and girls, almost no back flow. | (43) |
| | | Underwear side | Advantageous for both boys and girls, high absorption speed. | (44) |
| | Near to back | Near to center | Advantageous for few month-old-babies e.g., newborn babies, less SAP discomfort. | (45) |
| | | Uniform | Advantageous for few month-old-babies e.g., newborn babies, good balance of absorption performance. | (46) |
| | | Skin side | Advantageous for few' month-old-babies e.g., newborn babies, almost no back flow. | (47) |
| | | Underwear side | Advantageous for few month-old-babies e.g., newborn babies, high absorption speed. | (48) |

## Claims

1. A disposable diaper comprising a lower layer absorber and an upper layer absorber provided thereon,
wherein the upper layer absorber has a passage hole for loose stool,
at least one of the lower layer absorber and the upper layer absorber is formed by a fibber aggregate formed by opening tow.

2. The disposable diaper of claim 1, wherein at least the lower layer absorber is formed by a fibber aggregate formed by opening tow.

3. The disposable diaper of claim 1 or claim 2, wherein at least the upper layer absorber is formed by a fibber aggregate formed by opening tow.

4. The disposable diaper of claim 1 comprising a top sheet composed of a perforated sheet capable of passing loose stool and a body liquid impermeable sheet, wherein the upper layer absorber and the lower layer absorber are provided between the top sheet and the body liquid impermeable sheet.

5. The disposable diaper of any one of claims 1 to 4, wherein the fiber aggregate formed by opening tow has a fiber density of 0.0075 g/cm³ or less when the thickness is set to 10 mm.

6. The disposable diaper of any one of claims 1 to 5, wherein the fiber aggregate formed by opening tow contains a super absorbent polymer particle, and a holding sheet is provided on the back surface side of the lower layer absorber.
